# EUROPEAN PATENT APPLICATION

(11) **EP 0 862 914 A2**
(43) Date of publication of application: **09.09.1998**
(21) Application number: 98870031.6
(22) Date of filing: 18.02.1998
(51) Int. Cl.: A61K 7/48

(54) **Composition for the treatment and prevention of the formation of comedones**

(30) Priority: 18.02.1997 BE 9700149
(71) Applicant: "Pannoc Chemie N.V.", 2250 Olen (BE)
(72) Inventor: Spaas, Guido, 2250 Olen (BE)
(74) Representative: Quintelier, Claude

(57) **Abstract**

This invention relates to a composition for the treatment and prevention of the formation of comedones, which composition contains a gel and linoleic acid , whereby the linoleic acid is present as linoleic acid containing safflower oil. The composition preferably contains 5-20 wt % of safflower oil and 2.5-20 wt % of linoleic acid . The invention also relates to a process for the preparation of said composition, and the use of said composition in cosmetic applications.

## Description

This invention relates to a composition for the treatment and prevention of the formation of comedones, said composition containing a gel and linoleic acid.

Such compositions are applied topically to the skin, in order to remove existing comedones and to prevent the formation of new comedones.

Comedones (white spots and black spots, also called blackheads) are formed on the skin by a disturbed keratinization in the sebaceous gland ducts. The sebaceous gland ducts form the connection between the sebaceous glands and the outside world. The disturbed keratinization in the sebaceous gland ducts may be partly determined by hormones. The bonding together of substances present in the sebaceous glands, for example rejected cells, dirt, grease, etc., causes an accumulation in the sebaceous gland duct, which hampers the removal to the skin surface of substances which are located deeper in the sebaceous gland duct. As a result thereof, a comedo or plug may be formed in the sebaceous gland duct. The bacteria present in the sebaceous gland ducts are capable of decomposing fats to free fatty acids. This may irritate the skin cells in the vicinity of the sebaceous gland duct. Such an irritation may cause an inflammation, possibly followed by the formation of acne with papular and pustular, and finally acne lesions.

The composition referred to in the preamble contains approximately 1.75 wt % of linoleic acid and is known from TdB, no. 19, April-May, 1995. The linoleic acid present in the composition is capable of stimulating the metabolism in the sebaceous gland ducts, which allows to reduce the formation of comedones.

However, this composition has the disadvantage of being inadequately effective and of inadequately being capable of preventing the development of comedones. By applying above said composition topically, the formation of comedones can be reduced to some, but inadequate extent.

It is the aim of this invention to provide a composition allowing to prevent the formation of comedones almost completely.

This is achieved according to the invention because the linoleic acid is present as linoleic acid containing safflower oil.

Surprisingly, it has been found that the application of at least 500 mg of linoleic acid per cm² of skin surface allows to prevent the formation of comedones almost completely. If smaller quantities of linoleic acid are applied to the skin, the formation of comedones can only partly be prevented.

Surprisingly, it has also been found that safflower oil is particularly well incorporated by the skin and very rarely causes allergic reactions.

Because safflower oil mostly contains approximately 70 % of linoleic acid, only a small quantity of safflower oil has to be added to the composition, in order to obtain a composition having an adequate linoleic acid content. Because safflower oil moreover is particularly well incorporated by the skin, a relatively small quantity of safflower oil is sufficient to allow the application of 500 mg of linoleic acid per cm² of skin surface.

Moreover, safflower oii is relatively inexpensive, so that it is possible according to the invention to obtain an efficient composition for the prevention of comedones in an economical way.

Safflower oil is a natural vegetable oil which is frequently added to nutrients, as a source of unsaturated fatty acids, for example for margarines. It is not common practice to use safflower oil in cosmetic applications.

The composition according to the invention preferably contains 5-20 wt %, more preferably 7-15 wt % of safflower oil.

Linoleic acid may also be added to the composition in other ways, for example in order to further increase the linoleic acid content of the composition. Linoleic acid may be added as a free fatty acid. In that case, also an adequate quantity of a stabiliser should be added to the composition, to prevent as much as possible oxidation of the linoleic acid. Examples of suitable stabilizers are: tocopheryl acetate, retinyl palmitate, ascorbyl palmitate, ascorbic acid.

Linoleic acid may also be added to the composition in the form of a mixture. Linoleic acid is preferably added to the composition in the form of an oil with a high linoleic acid content. Examples of suitable oils are safflower oil, palmitic oil and myristic oil.

The quantity of linoleic acid in the composition is preferably 20 wt % at the most. This allows to apply a maximum of 4 mg per cm² of skin surface, and to prevent skin irritation as a result of an excess of linoleic acid.

Because linoleic acid is very rapidly oxidized upon contact with air, only a part of the linoleic acid applied to the skin in the form of linoleic acid will be incorporated by the sebaceous gland ducts. Moreover, only a part of the topically applied composition is resorbed by the sebaceous glands. Another part remains on the skin surface.

Because the composition contains at least 2.5 wt % of linoleic acid, it is possible to apply at least 500 mg of linoleic acid per cm² of skin surface, and to prevent largely the formation of comedones.

Linoleic acid is a poly-unsaturated fatty acid. Three isomers are known, namely linoleic acid with two double bonds, and α and γ linoleic acid , each of which contains three double bonds, in different locations in the structural formula. In principle, said linoleic acid isomers may all be applied according to the invention. According to the invention, preferably linoleic acid with two double bonds is applied, corresponding to the following chemical formula (I):

CH₃(CH₂)₃-(CH₂CH=CH)₂-CH₂)₇-COOH (I)

Surprisingly, it has namely been found that linoleic acid which answers to the above mentioned formula, is particularly suitable for the prevention of the formation of comedones; α and γ linoleic acid, on the other hand, are mainly used in the treatment of atopical dermatitis.

The composition according to the invention preferably contains 2.5-10 wt % of linoleic acid .

The composition according to the invention preferably also contains 1-10 wt % of lauromacrogol.

By the addition of lauromacrogol, it is possible to increase both the rate at which the composition is resorbed by the skin, and the quantity which is resorbed by the skin. In such a way, a composition with an increased efficiency for the prevention of comedones can be obtained.

If smaller quantities of lauromacrogol are added, the composition will only nenetrate to a small extent into the sebaceous gland ducts, and will result in a less efficient control of comedones. If larger quantities of lauromacrogol are added, skin irritation may occur.

The composition according to the invention preferably contains an anti-oxidant and a preservative.

The composition according to the invention preferably contains the following components: 0.1-1 wt % of vitamin E, 0.05-0.25 wt % of imidazolidic urea, 5-20 wt % of safflower oil, and 80-95 wt % of a gel, said gel containing the following components: carbomerum 940, preferably approximately 1-4 wt %; propylene glycol, preferably approximately 5 wt %; an agent to increase the pH, for example Neutrol TE; methylparaben, propylparaben and deionized water.

Such a composition, before being handled, is mostly packaged. This allows to preserve the composition sealed off from air.

Vitamin E is added to the composition as an anti-oxidant, in order to prevent linoleic acid, among others, from being already oxidized, before the package in which the composition is preserved, is opened. Imidazolidic urea is added as a preservative, so that a packaged composition can be preserved for at least 18 months. If larger quantities of safflower oil are added, it is not possible to obtain an emulsion, which makes use of the gel uncomfortable.

The composition according to the invention has preferably the form of a gel and is mainly built up of aqueous components. The composition according to the invention may also be manufactured in the form of a cream or a lotion.

The invention also relates to a process for the preparation of a composition to prevent the formation of comedones, in which to a gel containing the following components: carbomerum 940, preferably approximately 1-4 wt %; propylene glycol, preferably approximately 5 wt %; an agent to increase the pH, preferably neutrol TE; methylparaben, propylparaben and deionized water, in a nitrogen atmosphere, 0.1-1 wt % of vitamin E is added, after which 5-20 wt % of safflower oil is added in a nitrogen atmosphere, and the mixture is stirred.

The invention also relates to the use of the above described composition and the composition obtained with the above described process, for cosmetic applications, to prevent comedones.

The invention is further elucidated by means of the examples and comparative experiments below.

### Example 1

A composition was prepared, containing the following components in the indicated quantities: carbomerum 940, 1.15 wt %; propylene glycol, 5 wt %; neutrol TE, 1.70 wt %; methylparaben, 0.18 wt %; propylparaben, 0.02 wt %; deionized water, 78.71 wt %; vitamin E, 0.5 %; lauromacrogol, 6 wt %; and 10 wt % of safflower oil.

The face of 20 patients was treated during a period of 30 days with the above described composition. The quantity of comedones in the face decreased with 24.8 %.

### Comparative experiment A

The face of 20 patients was treated during a period of 30 days with the known composition, which does not contain linoleic acid . The quantity of comedones in the face increased with 3.25 %.

## Claims

1. Composition for the treatment and prevention of the formation of comedones, said composition containing a gel and linoleic acid , characterized in that the linoleic acid is present as linoleic acid containing safflower oil.

2. Composition according to claim 1, characterized in that the composition contains 5-20 wt %, preferably 7-15 wt % of safflower oil.

3. Composition according to any one of claims 1 or 2, characterized in that the composition contains 2.5-20 wt % of linoleic acid.

4. Composition according to claim 3, characterized in that the composition contains 2.5-10 wt % of linoleic acid .

5. Composition acording to any one of claims 1-4, characterized in that the linoleic acid corresponds to the chemical formula
CH₃(CH₂)₃-(CH₂CH=CH)₂-CH₂)₇-COOH (I)

6. Composition according to any one of claims 1-4, characterized in that the composition also contains 1-10 wt % of lauromacrogol.

7. Composition according to any one of claims 3-6, characterized in that the composition contains the following components: 0.1-1 wt % of vitamin E, 0.05-0.25 wt % of imidazolidic urea, 5-20 wt % of safflower oil, and 80-95 wt % of a gel, which gel contains the following components: carbomerum 940, preferably approximately 1-4 wt %; propylene glycol, preferably approximately 5 wt %; neutrol TE, methylparaben, propylparaben and deionized water.

8. Process for the preparation of a composition for the treatment and prevention of the formation of comedones.
